# NEW EUROPEAN PATENT SPECIFICATION

(11) **EP 1 916 989 B2**
(45) Date of publication and mention of the opposition decision: **12.03.2014**
(45) Mention of the grant of the patent: 06.10.2010
(21) Application number: 06828795.2
(22) Date of filing: 14.07.2006
(51) Int. Cl.: A61K 8/37, A61K 8/92, A61Q 1/10, A61Q 5/06

(54) **PREPARATION, IN PARTICULAR COSMETIC PREPARATION, PROCESS FOR THE PRODUCTION THEREOF, AND USE THEREOF**
ZUBEREITUNG, INSBESONDERE EINE KOSMETISCHE ZUBEREITUNG, EIN VERFAHREN FÜR DIE HERSTELLUNG DERSELBEN UND DIE VERWENDUNG DERSELBEN
PRÉPARATION, EN PARTICULIER PRÉPARATION COSMÉTIQUE, PROCÉDÉ DE PRODUCTION DE LADITE PRÉPARATION ET APPLICATIONS

(30) Priority: 14.07.2005 DE 102005033520
(43) Date of publication of application: 07.05.2008
(73) Proprietor: Schwan-STABILO Cosmetics GmbH & Co. KG, 90562 Heroldsberg (DE)
(72) Inventor: ZECH, Christina, 86916 Kaufering (DE); SWISTOWSKI, Azra, 90491 Nürnberg (DE)
(74) Representative: Leissler-Gerstl, Gabriele
(86) International application number: PCT/EP2006/006913
(87) International publication number: WO 2007/031139

(56) References cited:
- EP-A2- 1 172 078
- WO-A-2005/018599
- WO-A1-02//098378
- WO-A2-01/45662
- JP-A- 5 302 065
- JP-A- 57 155 254

## Description

The invention concerns a cosmetic preparation, preferably in liquid or pasty form, for use on the skin, on semi-mucous membranes, on mucous membranes and in particular on keratinic materials such as hair, eyelashes and eyebrows, in particular for shaping, decorating, colouring and improving the appearance thereof and for caring for the skin and the skin appendages. Preparations of that kind are used for example for shaping and colouring in particular the eyelashes and hair; such a preparation is then referred to as 'mascara'.

Basically the preparations according to the invention, with suitable adjustment and colouring, can also be used as makeup, concealer, camouflage, eyeshadow, eyeliner, lipliner, rouge, lip rouge, lip gloss, sun protection agent, sun blocker, temporary tattoo, coloured effect sun protection for surfers and the like.

Preparations, in particular cosmetic preparations, with which keratinic material, for example eyelashes or hair can be shaped and coloured, have already long been known. Improvements have repeatedly been developed in order to satisfy the many different demands which are to be made on such preparations. Compositions which are applied to hair or eyelashes are on the one hand to provide for good colouring of the hair or eyelashes, but on the other hand they should not transfer on to the surrounding area or other articles such as for example textiles. They should adhere well to hair or eyelashes but not permanently colour them and they should also be easy to remove again. It should be possible for them to be applied to the hair and eyelashes easily and uniformly, in particular they should impart an attractive shape to the eyelashes and also retain that shape after drying. For that purpose the compositions should be basically pasty but nonetheless of such low viscosity that they can be applied well and uniformly, but on the other hand they should dry quickly, so that the material is not smudged and eyelashes cannot stick together during the drying phase. In addition the preparations should be so stable that they can be stored over a prolonged period of time under ambient conditions. The ingredients thereof should therefore neither separate nor sediment.

To resolve the above-mentioned, in part contradictory demands the most widely varying forms of preparation and ingredients have been proposed. Thus mascara preparations are also known in the form of what is referred to as 'block mascara' or 'cake mascara' - a solid form of preparation from which the material is removed by means of a moistened brush - , in the form of 'emulsion mascara', in the form of O/W or W/O emulsions, or in the form of a solvent-based mascara. The use of a 'block mascara' is complicated if no water is available for moistening the brush or the material and another common name - namely 'spit mascara' - shows that alternative forms of use can certainly entail serious microbiologically induced risks in terms of product and user.

Other common mascaras contain a wax component which provides for adhesion to the hair or the eyelash, a film-forming agent which provides for an attractive curved shape for the eyelash and for retaining that shape, and binding agent for adjusting the viscosity in a suitable range. A mascara of that kind then always contains an aqueous phase and a lipid phase. Then - due to the type involved - at least one emulsifier must always be used in such an 'emulsion mascara' in order to keep the continuous phase in intimate relationship with the dispersed phase. A disadvantage with such a product is generally that, after drying on the eyelashes, it can be dissolved again due to moisture from the environment, for example water, rain, perspiration and in particular the quite slightly alkaline tear fluid, whereby the durability of such preparations is reduced. The use of water-soluble polymers or polymers which can be dispersed in water also involves detrimental effects here. An example of such a polymer-bearing product is to be found in W Umbach, 'Kosmetik', Georg Thieme Verlag, Stuttgart, 1988, page 101. Other examples are to be found in the standard works in cosmetics literature, for example H Janistyn, 'Handbuch der Kosmetika und Riechstoffe', volume 3, Hüthig-Verlag, 2nd edition, 1973, on pages 855-860 or G A Nowak, 'Die kosmetischen Präparate', Verlag Ziolkowsky, 3rd edition, 1984, pages 748-751.

In addition for example US No 6 210 692 describes cosmetic emulsions which are suitable inter alia as mascara and which are made up of an aqueous phase and a lipid phase, which, for stabilisation purposes, contain two different binding agents, namely a hydrophilic thickening agent and a polysaccharide ether, in which respect the use of such thickening agents which form gels with the aqueous phase is to be avoided. Those emulsions are said to be particularly suitable for caring for and treating dry and sensitive skin. In addition US No 6 325 994 describes a cosmetic composition which is suitable in particular as mascara and which comprises a lipophilic phase with fat and wax, as well as a lipophilic polymer, that lipophilic polymer being a specific acrylate-methacrylate copolymer. That composition can also occur in the form of an emulsion with an aqueous phase. Furthermore that composition may contain conventional additives such as colouring agents, pigments and fillers. Although that composition is intended to be water-resistant and very durable it is still not satisfactory in every respect.

The comparable compositions described in the above-mentioned literature usually contain lipids such as for example waxes and oils, emulsifiers, binding agents and colouring agents and they occur in emulsion form - either as an oil-in-water emulsion or a water-in-oil emulsion. The known preparations however suffer from at least one of the disadvantages referred to hereinafter. Many of the known emulsions are not sufficiently stable to be processed in the heated condition. For cosmetic compositions however a heat treatment may be required, which then necessitates specific and complicated and expensive treatment stages. Because of their nature, it is frequently difficult to incorporate colouring agents, in particular pigments, in such a way that they remain stable in the composition without settling out or forming lumps. Compositions which have good adhesion to keratinic materials are often difficult to remove therefrom again. In particular water-resistant and tear-resistant compositions can no longer be removed by simple washing with water and they then require the use of special 'makeup removers'.

In addition, a particular disadvantage with compositions of that kind is that, after drying on eyelashes or hair, they often form a brittle coating which can tear and flake off. In the case of hair mascara, that effect can certainly be desired because such a preparation, in the form of temporary hair colouring, can later be easily removed again by brushing it out - if however fine particles flake off the eyelashes, they can land in the eye and result in mechanical irritation, or for example in the case of ladies wearing contact lenses, it can also discolour or even worse, scratch the lenses; however the region under the eye can also be coloured (known as what is referred to as the 'panda bear effect').

EP 1 172 078 A2 discloses mascara compositions with improved wear characteristics like long lasting effect and curling properties. To enhance the water resistance and long wear characteristics film formers are used. Suitable film formers include PVP and PVP copolymers. Example 5 discloses a mascara composition in form of an O/W-emulsion comprising as emulsifying agent sorbitane sesquioleate. To enhance the wear characteristics PVP copolymer is used.

Solvent-bearing mascara preparations are also known, which contain only little or no water at all. Polar solvents such as ethanol, propan-1-ol, propan-2-ol, acetone, methylethylketone, ethylacetate, tetrahydrofuran, dichloromethane and the like are admittedly recommended in earlier technical literature - in that respect they automatically rule themselves out by virtue of their odour, their severely irritating effect on skin and mucous membranes and lack of environmental compatibility. Ethanol can at best be used in amounts below about 15% by volume. Non-polar isoparaffins have admittedly already been used in the past - in regard to some thereof, a point which tells against them is the aromatics content, which excludes them for cosmetic uses and the easy flammability of the low-boiling isoparaffins, whereby specific precautions are advised in the production of preparations of that kind. If however exclusively or predominantly higher-boiling C₁₁/C₁₂ isoparaffins are chosen, preparations which adhere very well to skin and hair are admittedly obtained, which have very good transfer resistance - however the odour is found to be unpleasant. A further disadvantage is also that diffusion problems and stress crack corrosion can occur when using containers of many kinds of polypropylene. Moreover, higher amounts of isoparaffins can cause condensation which is very inconvenient adjacent to or in the eyes.

EP 1 172 078 A2 discloses mascara compositions with improved wear characteristics like long lasting effect and curling properties. To enhance the water resistance and long wear characteristics film formers are used. Suitable film formers include PVP and PVP copolymers. Example 5 discloses a mascara composition in form of an O/W-emulsion comprising as emulsifying agent sorbitane sesquioleate. To enhance the wear characteristics PVP copolymer is used.

Therefore the inventors of the present application set themselves the object of developing a product which overcomes the disadvantages of the state of the art and which in particular is simple to manufacture and which can also be safely and reproducibly manufactured without apparatuses having to be especially provided. The invention also seeks to provide a product into which pigments can be easily incorporated and remain stable therein, wherein the product also affords good uniform colouring and nonetheless is not overburdened with pigments. A further object of the invention was to provide a product which is free of non-ionogenic emulsifiers based on polyoxyethylene (PEG) or polyoxypropylene (PPG) as they are judged to be a source of irritation particularly in the proximity of the eye. A further object of the invention was to develop a product which is stable at elevated temperature and which can therefore be heated without separation or destruction and which at elevated temperature is of such a viscosity that it can be easily filled into containers. In addition the aim is to provide that the product can be applied easily and uniformly, it adheres for a prolonged period of time, it extends the eyelashes and imparts bulk and volume to the eyelashes. In addition the product is to be of such a consistency that application mistakes can be removed and later corrections are possible and that even after hours makeup touching-up is still possible in order to further improve the appearance without the first application having to be removed beforehand. At the same time the invention aims to provide that the product according to the invention does not transfer on to eyelid surfaces, it is water-resistant and tear-resistant, it does not flake off or crumble away after drying and it can nonetheless be easily removed again with normal means, that is to say generally with warm water or soap or makeup removal cloths.

All those objects are attained by a preparation as defined in the claims. In particular in accordance with the invention there is provided a cosmetic preparation, which is in the form of an oil-in-water emulsion (O/W emulsion) and which besides usual cosmetic ingredients contains at least one wax component, a specific emulsifier and a PVP copolymer, wherein the ratio of emulsifier to PVP copolymer is 10: 1 to 3:1. Preferably it additionally contains at least one fatty acid, a film-forming agent, a polyvalent alcohol and a hydrocolloid as a gel component. The emulsifier according to the invention is a non-ionogenic emulsifier which has no PEG or PPG residues, namely sorbitan olivate.

It was surprisingly now found that a preparation with unusual properties can be obtained if the preparation is in the form of an O/W emulsion and contains the ingredients defined in the claims, in particular a specific emulsifier. It was surprisingly found that the emulsifier, although it is to be allocated to the class of W/O emulsifiers, can impart a very high level of stability to the O/W emulsion. Furthermore, the preparation can tolerate very high and very low levels of pigments. It is this property that makes the preparation versatile and suited for different requirements.

The ingredients of the preparation according to the invention are described in greater detail hereinafter.

The preparation according to the invention is in the form of an O/W emulsion which has an aqueous phase and a lipid phase. The aqueous phase can be formed from water or an aqueous medium, wherein the aqueous medium can be a mixture of water and a solvent which is compatible with water such as a low alcohol. Preferably the aqueous medium comprises water.

The lipid phase is formed from the hydrophobic constituents and has in particular a wax component. A specific emulsifier provides for the stability of both phases in the emulsion. The emulsifier specifically used is sorbitan olivate

The emulsifier according to the invention is the monoester of a sorbitol. Compounds of that kind are commercially available under the designation 'sorbitan ester'. Sorbitan olivate has been found to be particularly suitable, among the sorbitan esters, that is to say a sorbitol which is monoesterified with fatty acids as are present in olive oil, that is to say saturated fatty acids with 16 to 18 C-atoms.

The temperature stability of the emulsion according to the invention can be further improved if, besides the emulsifier according to the invention, the lipid phase contains a fatty acid whose chain length corresponds to the fatty acid present in esterified form in the emulsifier, preferably stearic acid is contained in the lipid phase. The use of the specific emulsifier, in particular in combination with the fatty acid which is preferably at least partially neutralised leads to very stable W/O emulsions with particularly good temperature stability. In addition colouring agents, in particular pigments, can be very easily and uniformly distributed in an emulsion of that kind and then no longer migrate out of that material so that the applied material no longer runs immediately, even upon contact with water, perspiration or tear fluid.

A further essential constituent of the O/W emulsion according to the invention is the wax component which forms the structure-imparting proportion of the lipid phase. The wax component comprises at least one wax and possibly additionally at least one fat and/or oil which can respectively be of vegetable, animal, mineral or synthetic origin. In addition the composition can contain at least one additional emulsifier and at least one co-emulsifier to facilitate processing of the wax component to afford an emulsion. In order to achieve a result which is particularly satisfactory from the aesthetic point of view, the wax component can additionally contain a polyvinylpyrrolidone copolymer.

The wax component imparts the desired consistency to the material and makes the composition water-resistant and tear-resistant. For that purpose the wax component can be made up from fat-like, oil-like and wax-like raw materials which can be fluid, pasty or solid at room temperature - that is to say at 23°C +/- 2°C. Preferably a combination comprising at least one wax and at least one oil is used for adjusting the optimum consistency, in which respect the term oil is used to denote a hydrophobic substance which is liquid at room temperature.

Preferably at least one wax which is solid at room temperature is used. The waxes are selected from those of vegetable, animal, mineral or synthetic origin or hybrids. Waxes with a dropping point of between 45 and 200°C and of a hardness of between 2 and 40 are particularly preferred. Waxes with a dropping point below 45°C can cause problems in terms of storage capability while waxes with a dropping point above 200°C are in part difficult in terms of processing. The dropping point is in that respect the temperature point at which the wax begins to drop. The dropping point of the wax can be determined for example in per se known manner insofar as the wax is definedly heated in a heatable apparatus with a lower opening and the temperature at which the first drop issues is determined. The hardness of the waxes is determined using the needle penetration method. The operation of determining the wax hardness is effected in accordance with the American Standard ASTM D5: at a temperature of 25°C a needle with a defined cone and weighing 2.5 g which is loaded with a weight of 47.5 g is caused to penetrate into a flat surface of a test body. The hardness value is determined as the depth of penetration in tenths of a millimeter after 5 seconds.

The waxes usually employed in cosmetics are suitable for the wax component used according to the invention, in particular vegetable waxes such as carnauba and candelilla wax, ouricuri wax, Japan wax, cotton wax, rice wax, flower waxes and/or fruit waxes such as orange flower wax, orange wax, jasmine wax or apple wax, hydrated jojoba oil; animal waxes such as beeswax, modified beeswax, inter alia so-called 'cera bellina' (polyglyceryl-3 beeswax), lanolin waxes, insect waxes and shellac wax; mineral waxes such as montan waxes, ozocerite, paraffin waxes and microcrystalline waxes, as well as synthetic waxes such as Fischer-Tropsch waxes, wax-polymer hybrids, polyethylene waxes, silicone waxes, long-chain esters such as cetyl palmitate, cetyl stearate, behenyl stearate, behenyl oleate, behenyl behenate, acelaic acid dioleylester, acelaic acid dibehenylester and mixtures of all the listed waxes. Vegetable waxes and synthetic waxes as well as in particular mixtures thereof are particularly preferred.

The wax component preferably includes at least one oil or fat in order to adjust the viscosity of the preparation to the desired range and to impart malleability and adhesiveness to the preparation. Considered for that purposes are oils and fats which can be of vegetable, animal, mineral or synthetic origin and which can also be used in hydrated or modified condition. Examples of suitable oils and fats are rapeseed oil, sunflower oil, sesame oil, ground nut oil, thistle oil, coconut oil, hydrogenated coconut oil, castor oil, hydrogenated castor oil, neat's foot oil, beef tallow, isopropyl myristate, isopropyl palmitate, isopropyl stearate, butyl stearate, isobutyl stearate, isostearyl-isostearate, oleyloeate, oleylerucate, diethyl sebacate, hexyl laurate, dibutyl adipate, caprylic/capric triglycerides and similar synthetic triglycerides, paraffin oils, polybutene, squalane, squalene, and mixtures thereof, synthetic esters, non-volatile silicone oils such as dimethicone (dimethylpolysiloxane), alkyl dimethicone whose alkyl residues can have chain lengths of between 10 and 36 carbon atoms, bis-PEG/PPG-16/16 PEG/PPG-16/16 dimethicone, diphenyl dimethicone, phenyl trimethicone, cyclomethicone, as well as non-volatile paraffins.

In addition a solvent or solubilisation agent may be included for better compatibilisation or dissolution in the wax component. Mention will be made here by way of example of volatile silicone oils as well as volatile paraffins and/or isoparaffins. Linear volatile silicone oils such as hexamethyl disiloxane, octamethyl trisiloxane, decamethyl tetrasiloxane and/or decamethyl pentasiloxane and/or cyclic silicone oils such as hexamethyl cyclotriloxane, octamethyl cyclotetrasiloxane, decamethyl cyclopentasiloxane and/or decamethyl cyclohexasiloxane can be considered. Among the volatile paraffins and isoparaffins, mention is to be made of those with 10 to 16 carbon atoms, in particular decane, isodecane, dodecane, isododecane, tetradecane and/or isotetradecane. In that respect C₁₁/C₁₂ isoparaffin is particularly advantageous. The isoparaffins are used in minor amounts. In that respect the use of mixtures of the specified substances is particularly advantageous.

The wax component according to the invention therefore frequently contains, besides the wax, a non-volatile oil or fat and a volatile oil as a solvent in order to impart various properties to the preparation. In one embodiment the wax component contains volatile constituents, in particular dimethicone and/or cyclomethicone and/or isoparaffins in a concentration of up to 20% by weight with respect to the total weight of the preparation. In an individual case the amounts involved can certainly slightly rise above or fall below the quantitative specifications set forth here and also hereinafter and in that case preparations according to the invention can nonetheless be obtained. For the man skilled in the relevant art, that would in no way be unexpected, in consideration of the large number of specified possible substances - he would know therefore that the fact of the amounts involved being above or below the specified quantities would not entail a departure from the scope of the above-specified invention.

The waxes, oils and fats are known per se and are used in the mixtures and amounts which are usually employed.

The wax component can in addition have still further constituents which are usually employed and which influence properties such as stability, viscosity, processibility and durability in known manner. Medium-chain to long-chain saturated, branched-chain or straight-chain fatty alcohols or fatty acids which as stated above are particularly adapted to the emulsifier are suitable for that purpose. Examples of suitable compounds are myristyl alcohol, cetyl alcohol, stearyl alcohol, behenyl alcohol, ceryl alcohol, myricyl alcohol, myristic acid, palmitic acid, stearic acid, arachic acid, behenic acid, cerotic acid, melissic acid or mixtures of the specified substances. These substances can have co-emulsifying properties when used with specific emulsifiers.

Preferably the above-described wax component is used in such a proportion that it forms 0.5 to 50% by weight, preferably 12 to 30% by weight, of the finished composition, the percentages always relating to the amount by weight in the finished composition. Within the wax component the proportion of the wax is preferably between 20 and 100% by weight and is particularly preferably between 60 and 90% by weight.

The oil or fat is usually added in such a proportion that the consistency of the material is in the desired range. Normally the proportion of the oils or fats in the wax component, depending on the other materials used, is between 20 and 80% by weight, in which respect the lower range is preferred, and with the proviso that the total of the percentages, with respect solely to the ingredients of the wax component, does not exceed 100% by weight.

In addition to the emulsifier according to the invention wax component may also contain a co-emulsifier or possibly also further emulsifiers. They promote emulsion formation and homogeneity of the preparation and stabilise the emulsion formed. Substances as mentioned above can be used.

Non-ionogenic emulsifiers can preferably be used here as anion-active, cationic or amphoteric emulsifiers result in irritation in the region of the eye because of their salt-like character, in particular if they come into prolonged contact with the mucous membranes of the eye. In particular the alkali salts of fatty acids - also as co-emulsifiers - have proven to be less suitable in that respect as the complete saponification thereof occurs at pH-values of between 9 and 10, which can result in irritation when acting for a prolonged period of time on the mucous membranes of the eye. Salts of organic bases with fatty acids are already formed in the weakly alkaline range, but certain organic bases can result in markedly perceptible and unpleasant secondary odours in a preparation. In accordance with more recent discoveries non-ionogenic emulsifiers which have PEG or PPG components can also result in irritation in the region of the eye.

The combination of the emulsifier according to the invention with saturated straight-chain or branched-chain fatty acids, at least partially neutralised with organic bases, with chain lengths of between 14 and 24 carbon atoms, has proven to be particularly advantageous, in which respect here stearic acid, isostearic acid or behenic acid, at least partially neutralised with aminomethyl propanol (AMP) or another amino base, has been found to be particularly advantageous. Stearin neutralised with triethanolamine, the commercially available mixture of palmitic and stearic acid, was also found to be suitable as a co-emulsifier, if certain odour problems are accepted in that respect, with a cheapening effect. In that respect, a quantitative ratio of sorbitan olivate to stearic acid, which is then at least partially neutralised with aminomethyl propanol or triethanolamine, of 3 to 1 to 1 to 3 was found to be particularly suitable.

The aqueous phase of the emulsion according to the invention preferably contains at least one moisturising agent for keeping it moist. In particular polyvalent alcohols but also vegetable extracts are used as the moisturising agent. Preferably the moisturising agent used is at least one divalent or polyvalent alcohol, wherein the alcohol optionally can also have further functional groups such as amino functions. Examples of suitable alcohols are propane diol, dipropylene glycol, tripropylene glycol, glycerin, diglycerin, triglycerin, butane diols, pentane diols, pentaerythritol, hexane diols, sorbitol, xylitol, mannitol, alditol, pantothenol or mixtures thereof. Their amount used in the preparation according to the invention is in that case between 0.3 and 10% by weight, preferably between 1 and 5% by weight. By virtue of their hygroscopic properties, the divalent or polyvalent alcohols usually serve in cosmetic preparations as moisture donors. In the preparation according to the invention, by virtue of their relatively very low evaporation rate, they serve as permanent plasticisers which reliably prevent the applied material from flaking or crumbling off. Because of their hygroscopic properties it is particularly surprising that they do not in any way adversely affect the water-resistance and tear-resistance of the preparation according to the invention.

In addition the emulsion according to the invention can contain further moisturising agents and plasticisers in order to make the material pleasant for the lady wearer after application. Here vegetable extracts which have softening and moisture-donating properties are suitable for that purpose. Bambusa vulgaris extract can be named here as an example

A further component which can be contained in the preparation according to the invention is a polymeric film-forming system. The film-forming system is usually formed from a polymer or a plurality of polymers which is/are present in a medium in the form of a dispersion or a solution, wherein the polymer or polymers forms or form a film upon the removal of that medium, that is to say the solvent or dispersing agent respectively. Depending on the kind of polymer the solid content is between 10 and 70% by weight, preferably between 20 and 50% by weight. In individual cases the polymers or polymer mixtures can also be in the form of solubilisable polymer powders. In the present application any system comprising film-forming agent and medium (also referred to as solubilisation agent, solvent or dispersing agent) is identified generally as 'dispersion' to avoid lack of clarity. As is known, the transitions between a dispersion and a solution are fluid in the field of polymers. Therefore the expression 'dispersion' also embraces solutions. Also included are systems in which the film-forming polymer was solubilised for example by the addition of an acid or base. The medium is preferably water.

Film-forming polymers are known per se and all those usually employed in cosmetic preparations can be used. The film-forming agent, together with the wax component, results in a very durable, water-resistant and tear-resistant coating for the keratinic material, for example for eyelashes, eyebrows, hair, hair piece, hair at the temples or beard.

The film-forming polymer can be produced by polyaddition, polycondensation or polymerisation or can be in the form of a polymer of natural origin. Particularly preferred in accordance with the invention are polyvinyl alcohol, PVP/alkene copolymer and functionalised polyurethanes, in particular polyurethanes hydrophilised by the incorporation of inorganic and/or organic acid esters. Sulphonate, sulphate, phosphonate, phosphate and carboxylate residues are to be listed by way of example as acid residues. The type of film-forming polymer has an influence on the water resistance of the final product.

A further essential component according to the invention is a PVP copolymer that is additionally used in the wax component. Said film-forming copolymer is present dissolved in a solvent or dispersed and can form a film after the application procedure. More specifically it has surprisingly been found that the use of the PVP copolymer such as PVP/hexadecene copolymer or PVP/eicosene copolymer or a mixture of the two copolymers in conjunction with the emulsifier according to the invention has an emulsion-stabilising effect. In that situation of use the copolymers act not only as a film-forming agent and dispersing additive for solids but surprisingly also as a co-emulsifier. In that respect a quantitative ratio between sorbitan olivate and PVP copolymer in the range of between 10 to 1 and 3 to 1 is selected.

The film-forming system is used in an amount which produces the desired effect. In that respect amounts in the range of 0.1 to 50% by weight are suitable. In order to achieve particularly advantageous properties in regard to durability, adhesion to the selected substrate and flexibility and shaping force, the amounts used are in the range of preferably 0.3 to 35% by weight and particularly preferably 1 to 25% by weight, wherein, in an individual case, the amount depends on the polymer used and the proportion and nature of the wax component and the optional gel component. The stronger the structure formed by the gel component or the stronger the respective film formed, the correspondingly smaller can be the amount of film-forming system. The optimum amount can be easily established by a man skilled in this art and optimised by routine tests. In this respect the quantitative specification relates to the system used, that is to say the amount of film-forming polymers and solubilisation agent.

The emulsion according to the invention can be further improved in terms of its texturing properties and its stability if the aqueous phase contains a gel component. Preferably the gel component used is a pre-swollen hydrocolloid which has a structure-forming action and at the same time stabilises the structure in such a way that after drying the composition forms a permanent, flexible and water-resistant and tear-resistant film which together with the film-forming agent and the wax component, retains the further ingredients of the preparation according to the invention in that composition in such a way that they are not leached out and cannot migrate or bleed out. The gel component therefore further contributes to providing that colouring agents and pigments but also further ingredients are stabilised and held fast in the composition.

As stated the gel component is formed from at least one pre-swollen hydrocolloid, swelling preferably being effected in water. It was found that a hydrocolloid in the swollen condition produces the desired lattice which then, in conjunction with the film-forming system, can accommodate and stabilise other ingredients. In connection with the present invention, those water-soluble, natural or synthetic polymers which form gels or viscous, in particular highly viscous solutions, in solvents, in particular aqueous systems, are to be identified as hydrocolloids. Examples of possible gel-forming agents are basically known in powder form and as thickening agents in cosmetic preparations - mention is to be made here by way of example of celluloses and cellulose derivatives, starch and starch derivatives, alginates, pectins, carrageenans, tragacanth, plant rubbers, polyvinyl alcohol, polyvinyl pyrrolidone and dextran and mixtures thereof. Hitherto however it was not realised that they can impart very advantageous properties to a cosmetic preparation, in the pre-swollen condition. In that respect, a combination of polyvinyl alcohol and hydroxyethyl cellulose has surprisingly been found to be particularly advantageous, if in that respect a quantitative ratio of between 10 to 1 and in particular 6 to 1 is adopted. It was found in that case that this combination of two hydrocolloids in the pre-swollen condition imparts structure, stability and volume to the preparation according to the invention. The use of the combination according to the invention of two pre-swollen hydrocolloids can even have the result that the volume of the preparation is scarcely reduced or not reduced at all, after drying. In that way the preparation according to the invention, even after drying, makes keratinic materials, in particular eyelashes, fuller and longer, imparts a curve thereto and thus enhances the aesthetic impression.

It was found that particularly good properties can be achieved if the hydrocolloid or hydrocolloids are added to the further constituents in the fully swollen condition, that is to say in the form of a stable aqueous gel or a highly viscous aqueous solution Those properties cannot be achieved if the gel-forming agent is allowed to swell in the preparation. Particularly good properties are achieved if the hydrocolloid or hydrocolloids is pre-swollen in water having ambient or higher temperature, preferably in heated water, particularly preferred in water at a temperature of between 40 and 90°C. For that purpose they are preferably added to the water and left therein until no further water absorption occurs. After complete swelling, prior to further processing, they are allowed to cool to a temperature preferably below 40°C, particularly preferably to room temperature. Due to the swelling effect the physical properties of the hydrocolloids are irreversibly changed, and that affords the desired structure. With that structure the fully swollen hydrocolloids are stable and storable and can therefore be stored for some time, for example some hours or even over still longer periods of time, prior to further processing.

To avoid microbial attack on the pre-swollen hydrocolloid system during storage, it is possible to add a preserving agent. Preserving agents for systems of that kind are known to the man skilled in the art. The preserving agents which are approved and suitable for foods and cosmetics can be employed for that purpose.

In addition a dispersing additive can also be added to the system. It was found that a combination of preserving agent and dispersing additive can synergistically enhance the anti-microbial effect. In that connection an addition of EDTA and salts thereof also has an advantageous effect as calcium ions are complexed in that way, which makes the cell membranes of micro-organisms more permeable for preserving agent. Benzyl alcohol, phenethyl alcohol, phenoxy ethanol, esters of p-hydroxybenzoic acid and salts thereof, IPBC, formaldehyde splitters such as imidazolidinyl urea or diazolidinyl urea, organic and organic aromatic carboxylic acids and mixtures of the specified substances can be mentioned as suitable preserving agents. The preserving agents and dispersing additives, if used, are employed in the amounts which are usual per se.

The preparation according to the invention may also contain still further usual cosmetic ingredients such as antioxidants, fragrances, vitamins, extracts, active substances, protein hydrolysates and other additives which influence certain desired properties. An important ingredient for cosmetic preparations, in particular for decorative cosmetic preparations, are colouring agents, that is to say dyes or pigments, in order to give a desirable colour impression. Besides those, it is also possible to use fillers in the form of preferably finely divided, inorganic or organic solids. It was found that the preparation according to the invention is suitable for stabilising both fillers such as talcum, kaolin, healing earth, smectite, bentonite, hectorite, montmorillonite, starch and modified starch, non-swelling starches, water-insoluble celluloses, polytetrafluoroethylene powder (Teflon), polyamide powder (Nylon), polyethylene powder, polypropylene powder, silk powder, silicon dioxide, boron nitride, mica, fine fiber material of silk, cotton, wool, linen, polyamide, polyacrylonitrile, polyesters, elastanes, viscose, artificial silk, cellulose or the like, insoluble metal soaps such as Mg-stearate, Ca-stearate, Sr-stearate or Zn-stearate or mixtures thereof and/or for including and dispersing inorganic or organic pigments and lakes of organic colouring agents in such a way that they cannot be leached out or emigrate or bleed out. All colouring agents which are known per se for cosmetics and preferably those which are approved for cosmetics by the respective national or regional legislation can be used as the colouring agents. Dyes are in that respect generally less advantageous because under some circumstances they can colour keratinic materials semi-permanently, that is to say over a period of several days; use thereof is however not basically excluded as a result, as that can certainly also be a desired effect. Preferably pigments are used for colouring the preparation according to the invention, in micronised form. In quite general terms the expression 'pigments' is used to denote white or coloured, inorganic or organic particles which are insoluble in water and the respective medium and which can colour and/or cloud a composition. This can involve white or coloured, inorganic or organic pigments of commercially available size or with particles sizes in the nanometer range, that is to say so-called 'nanopigments'. Ultrafine aluminium oxide, cerium oxide, titanium oxide, zinc oxide or mixtures thereof which have average particles sizes in the range of between 5 and 25 nm are to be mentioned in that area. The man skilled in the relevant art implements the choice of suitable colouring agents in accordance with the desired effects and possibly within the limits of statutory requirements. Examples of suitable inorganic and organic pigments are titanium dioxide, zinc oxide, barium sulphate, carbon black, carmine, yellow, red or black iron oxide, chromium oxide green, chromium hydroxide green, ultramarine, Berlin Blue (Ferric Blue), manganese violet. Examples of organic colouring agents are lakes of organic dyes such as aluminium, barium, calcium, potassium, strontium or zirconium lake and other substances which are well-known to the man skilled in the art or mixtures of the specified substances. In addition it is also possible to use pearlescent pigments and other 'glitter substances' such as titanium dioxide-coated mica and coloured mica coated with titanium dioxide and metal oxides, bismuth oxychloride, bismuth oxychloride coated with metal oxides, flake-form metal powders of aluminium, bronze, brass, copper, silver and gold. Also suitable are 'glitter substances' based on fine flake-form glasses which can possibly also be at least partially coated with titanium dioxide and/or coloured metal oxides, flake-form metal powders at least partially coated with other materials, plastic flakes at least partially coated with other materials, preferably PET flakes, solid solutions of dyes in solid plastic matrices, preferably polyester-3 or mixtures of the above-mentioned coloured substances. The last-mentioned solid solutions of dyes in plastic matrices which are distinguished by intensively lighting up in long-wave UV-light, so-called'black light', are commercially inexpensive and known for example under the INCI names 'Polyester-3, Red 22', 'Polyester-3, Red 28', 'Polyester-3, Yellow 10' or 'Polyester 3, Blue 1', see in that respect for example also WO 2004/108108. They can also be used in mixtures with each other, possibly also in conjunction with UV-active stilbene derivatives which are allowed for cosmetic uses and which are distinguished in long-wave UV-light by an intensive light-blue lighting effect or in any mixtures with the above-mentioned colouring agents which can also be in a form which is physically or covalently surface-coated with silicones, titanium triisostearate, or fatty acids, for example hydrophobised, according to the desired colour effects. That list is only by way of example and is in no way definitive.

The structure of that O/W emulsion according to the invention is so stabilised, in the solid and also the pasty condition, that no separation of the mixture occurs even at relatively high temperature. That affords the advantage that it can be heated without any problem and can be introduced into a container in the heated condition and thus at low viscosity, which facilitates and speeds up the production and filling process. Production of the preparation according to the invention is additionally simple and can be implemented easily and in a reliably reproducible fashion using simple available items of equipment. In that respect it is particularly advantageous to use a vacuum process installation which permits uniform de-aeration of the preparation for further improving storage stability.

The cosmetic preparation is particularly intended to be applied to keratinic material, in which respect consideration is given in particular to eyelashes, eyebrows, hair and hair pieces. It is also possible to use the preparation according to the invention for men for concealing hair which has turned grey at the temples or for dyeing beards. The preparation according to the invention is particularly suitable in the form of a mascara.

If the preparation according to the invention is used as mascara, generally those pigments are used, which result in the colours that are usually wanted, namely black, brown, blue, green, violet and grey. Nonetheless it is however also possible to produce preparations of other colours in order to fashionably accentuate and give high-contrast colouring to the eyelash tips or the eyelashes also overall with contrasting colours, for example light green, moss-green, yellow, orange or different shades of red. Particularly in the field of hair mascara, the man skilled in the art, based on the above-listed colouring agents, is presented with a wide range of possible options including for the sector of so-called 'effect makeup', for example in regard to colours for the consumers to visit a disco.

The amount of the finely divided solids phase comprising inorganic and/or organic fillers and inorganic and/or organic colouring agents is generally in the range of between 0.1 and 30% by weight, preferably between 1 and 20% by weight and particularly preferably between 5 and 15% by weight.

The colouring constituents do not necessarily have to be contained in the preparation according to the invention. It is also possible to have an embodiment in which colouring and structuring constituents are separate, wherein firstly the preparation according to the invention is applied without colouring ingredients to the keratinic material and then a formulation which contains the colouring agents is applied. It is also possible for in particular large-area 'glitter substances' which can be of average particle sizes in the range of about 50 to 500 µm to be suitably subsequently applied in dry form to the preparation according to the invention which is not coloured and when it has not yet dried.

The pH-value of the preparation according to the invention is preferably in the neutral to weakly basic range, that is to say in the range of tear fluid which has a pH-value of about 7.4. More strongly acid or basic compositions are unfavourable for use on the eye. In addition a pH-value outside the neutral and weakly basic range can lead to a change in constituents contained in the preparation. For example for organically neutralised fatty acid which can be used as a co-emulsifier and emulsion stabiliser, it is advantageous if the medium is neutral as it is only then that that compound can deploy its full effect. Optionally, to adjust and buffer the pH-value the preparation according to the invention also contains basic agents and/or buffering agents, for example amines and conventional buffer substances. Preferably the pH-value of the preparation is so adjusted that it is in the range of 7.0 to 8.5 and preferably 7.7 to 8.2.

A further ingredient which can be contained in the preparation according to the invention is a steroid or steroid derivative. It was found that particularly good properties are achieved when a steroid derivative is used in combination with the emulsifier according to the invention. Colophony is frequently contained in cosmetic preparations in the state of the art, for structuring purposes. Colophony however contains abietic acid which can irritate the eye and is therefore inappropriate precisely for preparations which are to be used on the eye in the form of mascara, eyeshadow or eyeliner. It was found however that steroids or steroid derivatives can produce a similar effect to colophony without suffering from the troublesome side-effects. Particularly suitable steroid derivatives are sterols and their hydrated derivatives and the vegetable extracts containing them. Phytosterols and phytostanols can be mentioned as examples of sterols and their hydrated derivatives. A preferred sterol is oryzanol. The non-saponifiable component which is obtained in oil production is suitable as a material which contains sterols and/or stanols. Candellila resin can also substitute colophony in the present preparations.

According to the invention therefore there is provided a preparation which is in the form of an emulsion and which combines particularly advantageous properties. The preparation can be easily produced, it leads to stable products, it can be easily subjected to further processing as it is stable in the hot condition. It produces a film which adheres for a long period of time without liberation of the ingredients contained therein. The film causes the keratin fibres to which it is applied to look full and gives them shape and curve - it 'curls' them therefore in an attractive and permanent fashion. At the desired time the preparation can also be easily removed again by applying water, possibly slightly heated and provided with a wetting agent, which results in swelling of the film whereby it can be easily removed again without leaving any residue. The product according to the invention however can equally well be removed again with a commercially available makeup remover or suitable cloth. The special combination of a specific emulsifier with the wax component and the aqueous phase forms a structured composition which adheres durably to the keratin fibre so that the result obtained is a homogeneous, bulky, flexible film which lasts for a long time.

A further subject of the invention is a process for the production of that preparation, an example thereof is a process as set forth in claim 29.

In a preferred embodiment the preparation of the present invention can be produced as follows:
I. a) an aqueous gel phase is prepared by preswelling at least one hydrocolloid in an aqueous medium, preferably at ambient temperature or higher temperature, more preferred at a temperature in the range of 40 to 90°C, water soluble or hydrophilic ingredients can be added; b) a wax phase is prepared by melting at least one wax together with hydrophobic ingredients, optionally a base is added; thereafter water the temperature of which has been adapted to the wax melt can be added; c) optionally a solid phase is prepared by mixing pigments, fillers and other particulate matter with water;
II. the aqueous gel phase and the wax phase are combined with thorough mixing whereby an emulsion is formed;
III. optionally the solid phase is added with stirring;
IV. finally temperatures sensitive ingredients such as antioxidants, fragrances, substances etc. are added;
V. the finished preparation can be filled in storage containers or, before or after cooling, can be directly filled into application devices.

The preparation of the aqueous gel phase is an important part of the method of the present invention. It is essential that the at least one hydrocolloid is preswollen in the aqueous medium. The preswelling is done until the at least one hydrocolloid does not uptake any further aqueous medium. In this form the hydrocolloid can be processed immediately or can be stored for further processing.

In the following one specific embodiment of the process is described:

To produce the preparation according to the invention, optionally the aqueous gel phase is prepared by the hydrocolloid or hydrocolloids being pre-swollen in a partial amount of the water required for production of the preparation. For that purpose preferably heated water which is advantageously at a temperature of 40 to 90°C and preferably 70 to 90°C is brought into contact with the hydrocolloids with agitation and then thereafter possibly allowed to cool and swell. In a preferred embodiment at least one divalent, polyvalent alcohol or a mixture of two or more of those alcohols is then added to that mixture. A preserving agent and/or a dispersing additive and/or the base required for at least partial neutralisation of the fatty acid can optionally also be added to that mixture - the base however can equally well be added to the remaining amount of water, which is still present, at a later time, that is to say immediately prior to production of the emulsion. The swollen gel is stable and can be stored even over prolonged periods of time so that it does not have to be subjected to further processing immediately.

The constituents of the wax phase are weighed out into a suitable vessel and heated together to 50 to 100°C, preferably 60 to 80°C. The remaining amount of water is adapted to the temperature of the melted wax component, and is added optionally with the necessary amount of base to the liquid wax component with vigorous agitation. To provide for intensive thorough mixing of emulsion which is being formed, a high-speed agitator is preferably used or the entire production procedure is implemented in an evacuatable process installation. Thereafter, the swollen gel phase is added and the composition is stirred for several minutes. The solids phase comprising pigments and optionally fillers is then added with agitation. Now the temperature-sensitive substances such as for example antioxidants, fragrance mixtures, finished polymer dispersions, volatile substances such as silicone oils or isoparaffins and the like can be added and the finished preparation can then be filled into fixedly closable storage containers either directly or after cooling. It is also possible to convey away directly to the filling operation and fill into suitable, commercially available application devices. The process according to the invention makes it possible for the preparation to be manufactured easily, in a well-reproducible fashion and rationally in large numbers. The filling operation while still in the heated condition in accordance with the process of the invention thus affords advantages both from the point of view of hygiene and also from the point of view of process engineering.

Conventional application devices for mascara - they are referred to hereinafter as 'containers' - usually have a receiving space comprising a transparent material or a material which is coloured with the colour of the material or the 'corporate colour' of the supplier, wherein the receiving space is provided with a releasably mounted closure on which there is disposed an inwardly extending stem carrying a brush for application of the material to be applied. Corresponding containers are commercially available and are sufficiently known to the man skilled in the art - here he only has to take account of the fact that the selected brush provides for a good wetting effect for the eyelashes and in so doing separates them and that the brush and the scraper portion and in addition the material of the container and the ingredients of the mascara preparation are well matched to each other. If a mascara preparation and an effect mascara are offered in combination, a packaging which can be combined from two individual units is then recommended or also a combination packaging in which one unit can be provided with a smaller volume and a smaller brush. As normally a person has more hair on the head than eyelashes, a larger container naturally has to be adopted for hair mascara. The preparation according to the invention, in particular the cosmetic preparation, is particularly well suited to coating keratinic materials. It is therefore preferably used for colouring and/or shaping eyelashes, eyebrows, hair, hair pieces, hair which has turned gray at the temples, beard hair and other keratinic materials. The preparation according to the invention is particularly suitable as mascara as it clings firmly to the eyelashes even after only one application, covers them well and uniformly, it does not migrate out, it does not come off and it does not bleed out, it does not crumble away and it does not flake off and it remains on the eyelash for a long time and in a durable state until it is removed again. As it can have a smaller proportion of solids than previously known products, it is forgiving in regard to mistakes in use and allows subsequent corrections. It can also be charged with a high amount of pigments without crumbling away after application. The composition is very versatile regarding the amount and ratio of pigments. The volatile component contained in the preparation according to the invention causes minimal surface dissolution of the film present on the eyelash and thus results in an intimate join between material which is already present there and freshly applied material. In that respect drying of the preparation takes place with something of a delay and in two steps as firstly the water and then the volatile component evaporates, which in turn is conducive to the 'curl' of the applied film.

A further subject of the invention is therefore the use of the preparation according to the invention for colouring and shaping keratinic material such as eyelashes, eyebrows, hair, hair pieces, hair which has turned gray at the temples, beard hair and other keratinic materials, in particular use as mascara.

### Embodiments

The invention is described in detail by the Examples hereinafter without however being restricted thereto. In that respect the amounts are given in percent by weight (% by weight), in each case with respect to the total weight of the preparation. The raw materials are identified by use of the 'INCI' names which are generally familiar to the man skilled in the relevant art - 'INCI' is the 'International Nomenclature of Cosmetic Ingredients'.

### Example 1 (pasty mascara preparation)

| (A) | |
|---|---|
| Aqua (freshly deionised water) | 51.380 |
| Polyvinyl alcohol | 6.000 |
| Hydroxyethyl cellulose | 1.300 |
| Sorbitol | 2.500 |
| Glycerin | 1.000 |
| Panthenol | 0.250 |
| Aminomethyl propanol | 0.550 |
| | |

| (B) | |
|---|---|
| Sorbitan olivate | 6.500 |
| Stearic acid | 4.000 |
| PVP/hexadecene copolymer | 1.500 |
| Copernicia cerifera (carnauba) | 3.500 |
| Oryza sativa (rice bran wax) | 4.500 |
| Isopropyl myristate and bambusa vulgaris extract | 0.200 |
| Bis-PEG/PPG-16/16 PEG/PPG-16/16 dimethicone | 0.500 |
| Caprylic/capric triglyceride | 0.300 |
| | |

| (C) | |
|---|---|
| Iron oxide black (C.I. No 77.499) | 9.500 |
| Tocopherol acetate | 0.350 |
| Fragrance | 0.250 |
| Dimethicone 1, 5 st | 4.500 |
| Polyurethane-2 | 1.000 |
| IPBC | 0.020 |
| Phenoxyethanol | 0.400 |

For the production procedure the above-specified amount of water is put into a sufficiently large vessel and heated to about 70°C. The polyvinyl alcohol and the hydroxyethyl cellulose are then added with vigorous agitation using a toothed ring agitator and agitated to a lump-free condition. Next sorbitol, glycerin and panthenol are added. Moreover, the aminomethyl propanol is added to the aqueous phase. IPBC and phenoxyethanol can possibly also be added. The constituents of phase (B) are heated in a separate vessel to about 85°C. The liquid phase (B) is added to the aqueous phase, the mixture is homogenised briefly and cooled to about 45°C. The mixture is now homogenised for some minutes, the solid phase is added, the mixture is homogenised once again briefly. The constituents which have not yet been added are now added, the pH-value is adjusted to between 7.2 and 7.4 and then the mixture is selectively either cooled to room temperature or the composition is filled in a condition of still being heated into sealingly closable storage containers or it is prepared for filling into conventional application devices. The product obtained is a deep black pasty preparation with a good sheen, which can be very easily and uniformly distributed on keratinic material, which has extremely good adhesion thereto and which is completely dried relatively quickly.

### Example 2 (pasty, colourless mascara preparation)

| (A) | |
|---|---|
| Aqua (water, freshly distilled) | 56.830 |
| Polyvinyl alcohol | 5.500 |
| Carrageenan | 1.500 |
| Mannitol | 2.000 |
| Propylene glycol (propan-1,2-diol) | 1.700 |
| Aminomethyl propanol | 0.650 |
| | |

| (B) | |
|---|---|
| Sorbitan olivate | 6.500 |
| Behenic acid | 4.200 |
| PVP/eicosene copolymer | 1.900 |
| Euphorbia cerifera (candellila wax) | 4.500 |
| Behenyl behenate | 4.000 |
| Bis-PEG/PPG-16/16 PEG/PPG-16/16 dimethicone | 0.500 |
| Octyldodecanol | 0.300 |
| | |

| (C) | |
|---|---|
| Tocopherol acetate | 0.350 |
| Fragrance | 0.250 |
| Isopropyl myristate and bambusa vulgaris extract | 0.200 |
| Dimethicone 1, 5 st | 6.500 |
| Polyurethane-2 | 2.200 |
| IPBC | 0.020 |
| Phenoxy ethanol | 0.400 |

Production and processing are effected in a similar manner to Example 1, with omission of the step of pigment incorporation. The result obtained is a shiny material which is colourless after drying and which can be applied to eyelashes to impart fullness and curl thereto. When applied to the hair, before the film dries, coarser particles of a 'glitter substance', for example coloured polyester particles, can be scattered thereonto or incorporated.

### Example 3 (pasty effect hair mascara)

| (A) | |
|---|---|
| Aqua (water, freshly distilled) | 50.750 |
| Polyvinyl alcohol | 5.000 |
| Tragacanth | 2.000 |
| Xylitol | 2.500 |
| Dipropylene glycol | 1.800 |
| Triethanolamine | 0.750 |
| | |

| (B) | |
|---|---|
| Sorbitan olivate | 5.500 |
| Isostearic acid | 4.500 |
| PVP/eicosene copolymer | 1.800 |
| Copernicia cerifera (carnauba) | 4.000 |
| Ouricuri wax | 3.500 |
| Bis-PEG/PPG-16/16 PEG/PPG-16/16 dimethicone | 0.600 |
| Butyl stearate | 0.400 |
| | |

| (C) | |
|---|---|
| Polyester-3, Red 28 | 5.500 |
| Titanium dioxide (C.I.-No 77891), nanopigment | 2.500 |
| Tocopherol acetate | 0.200 |
| Isopropyl myristate and bambusa vulgaris extract | 0.200 |
| Fragrance | 0.150 |
| Decamethylcyclopentasiloxane | 5.500 |
| Polurethane-2 | 2.200 |
| Methylparaben | 0.150 |
| Propylparaben | 0.100 |
| Phenoxyethanol | 0.400 |

Production is effected in a similar manner to Example 1. The result obtained is a shiny, magenta-coloured, pasty preparation which can be easily and uniformly applied to the hair and which is preferably suitable for colouring streaks. In long-wave UV light the preparation has intensive red-violet fluorescence.

### Example 4 (comparative example - mascara)

| (A) | |
|---|---|
| Aqua (water, freshly distilled) | 57.550 |
| Acacia Senegal gum | 2.000 |
| Hydroxyethyl cellulose | 1.300 |
| Polyvinyl alcohol | 4.000 |
| Triethanolamine | 0.650 |
| Panthenol | 0.250 |
| | |

| (B) | |
|---|---|
| Hydrogenated stearyl olive esters | 5.000 |
| Stearic acid | 3.000 |
| Palmitic acid | 2.500 |
| Oryza sativa (rice bran wax) | 4.500 |
| Copernica cerifera (carnauba) | 1.500 |
| Euphorbia cerifera (candelilla wax) | 2.000 |
| Dimethicone copolyol | 1.500 |
| Simethicone | 1.000 |
| | |

| (C) | |
|---|---|
| Black iron oxide (C.I.-No 77499) | 9.500 |
| Mica (C.I.-No 77891) | 1.500 |
| Methylparaben | 0.150 |
| Propylparaben | 0.100 |
| PPG-17/PDI/DMPA copolymer | 2.000 |

Production is effected in a similar manner to Example 1; the preparation however is cooled in conventional manner to room temperature after a partial amount discharged at 40°C, after cooling, exhibited a gritty structure. The result obtained is a deep black, pasty, shiny material which can be applied well and uniformly to the eyelashes and which clings firmly there. In an attempt to apply the material once again to an eyelash which had already been previously coloured with the same material, that results in an irregular appearance involving irregular structures. The material has a dank, musty odour of its own. Stored in a closed glass container over 24 hours at 50°C, the material assumes a gritty structure and at the edge exhibits syneresis and liquid deposits on the surface. The surface has a dull and very non-homogeneous effect. In a preservation stress test, after just a single inoculation, the material exhibited massive growth with mould and yeast.

## Claims

1. A cosmetic preparation, in the form of an O/W emulsion, which besides usual cosmetic ingredients contains a wax component, an emulsifier and a PVP copolymer, wherein the ratio of emulsifier to PVP copolymer is 10:1 1 to 3:1, and wherein the emulsifier is sorbitan olivate.

2. A cosmetic preparation according to claim 1 **characterised in that** the wax component contains one or more waxes which are solid at room temperature and have a dropping point of 45 to 200°C.

3. A cosmetic preparation according to claim 1 or claim 2 **characterised in that** the wax component contains one or more waxes which are of a hardness of 2 to 40, determined using the needle penetration method in accordance with ASTM D5.

4. A cosmetic preparation according to one of the preceding claims **characterised in that** the wax component additionally contains at least one fat and/or oil, at least one fatty acid, at least one polyvalent alcohol and/or at least one film-forming agent.

5. A cosmetic preparation according to one of the preceding claims **characterised in that** a fatty acid which corresponds to the fatty acid esterified in the emulsifier is present as co-emulsifier.

6. A cosmetic preparation according to claim 5 **characterised in that** the co-emulsifier is a fatty acid partially neutralised with an organic base.

7. A cosmetic preparation according to claim 6 **characterised in that** the organic base is aminomethyl propanol or triethanolamine or a mixture thereof.

8. A cosmetic preparation according to one of the preceding claims **characterised in that** the co-emulsifier is partially neutralised stearic acid.

9. A cosmetic preparation according to one of the preceding claims **characterised in that** the ratio of emulsifier to co-emulsifier is 3:1 to 1: 3.

10. A cosmetic preparation according to one of the preceding claims **characterised in that** the PVP copolymer is a PVP/alkene copolymer.

11. A cosmetic preparation according to one of the preceding claims **characterised in that** the PVP copolymer is a PVP/hexadecene copolymer or a PVP/eicosene copolymer or a mixture thereof.

12. A cosmetic preparation according to one of the preceding claims **characterised in that** at least one divalent or polyvalent alcohol is included.

13. A cosmetic preparation according to claim 12 **characterised in that** the divalent or polyvalent alcohol is selected from propane diol, dipropylene glycol, tripropylene glycol, glycerin, diglycerin, triglycerin, butane diols, pentaerythritol, hexane diols, sorbitol, xylitol, mannitol, alditol, pantothenol or mixtures thereof.

14. A cosmetic preparation, according to claim 13 **characterised in that** the amount of the at least divalent alcohol used is between 0.3 and 10% by weight, preferably between 1 and 5% by weight, with respect to the total weight of the preparation.

15. A cosmetic preparation, according to any of claims 4 to 14 **characterised in that** the film-forming agent is selected from polyvinyl alcohol, PVP/alkene copolymer or a functionalised polyurethane or mixtures thereof.

16. A cosmetic preparation, according to claim 15 **characterised in that** the functionalised polyurethane is a hydrophilised polyurethane into which inorganic and/or organic acid residues are incorporated.

17. A cosmetic preparation, according to claim 16 **characterised in that** the acid residues are sulphonate, sulphate, phosphonate, phosphate or carboxylate residues.

18. A cosmetic preparation, according to one of the preceding claims 15 to 17 **characterised in that** the functionalised polyurethane is polyurethane-2.

19. A cosmetic preparation, according to one of the preceding claims 14 to 18 **characterised in that** the film-forming agent is used in amounts of 0.1 to 50% by weight, preferably 0.3 to 35% by weight and particularly preferably in amounts of 1 to 25% by weight, with respect to the total weight of the preparation.

20. A cosmetic preparation according to one of the preceding claims **characterised in that** the aqueous phase contains a hydrocolloid as gel-forming agent.

21. A cosmetic preparation according to claim 20 **characterised in that** the hydrocolloid is selected from celluloses and cellulose derivatives, starch and starch derivatives, alginates, pectins, carrageenans, tragacanth, plant rubbers, polyvinyl alcohol, polyvinyl pyrrolidone, dextran and mixtures thereof.

22. A cosmetic preparation according to one of the preceding claims **characterised in that** the aqueous phase is a pre-swollen hydrocolloid selected from polyvinyl alcohol, hydroxyethyl cellulose, carrageenan or tragacanth.

23. A cosmetic preparation according to one of the preceding claims **characterised in that** the cosmetic preparation additionally contains a finely divided solids phase.

24. A cosmetic preparation according to claim 23 **characterised in that** the solids phase is contained in a proportion of 1 to 30 % by weight, preferably 1 to 20 % by weight and particularly preferably 5 to 15 % by weight.

25. A cosmetic preparation according to one of claims 23 or 24 **characterised in that** the finely divided solids phase comprises inorganic and/or organic fillers and/or fine fibre materials and/or inorganic and/or organic pigments and mixtures thereof.

26. A cosmetic preparation according to one of the preceding claims **characterised in that** a steroid derivative or a steroid-containing material is additionally included.

27. A cosmetic preparation according to claim 26 **characterised in that** phytosterol or stanol in free or esterified form is included as the steroid derivative.

28. A cosmetic preparation according to claim 27 **characterised in that** oryzanol or rice bran wax is included.

29. A process for the production of a cosmetic preparation according to any of claims 1 to 28, wherein
**I**. a) an aqueous gel phase is prepared by preswelling at least one hydrocolloid in an aqueous medium; water soluble or hydrophilic ingredients can be added; b) a wax phase is prepared by melting at least one wax together with hydrophobic ingredients, optionally a base is added; thereafter water the temperature of which has been adapted to the wax melt can be added; c) optionally a solid phase is prepared by mixing pigments, fillers and other particulate matter with water;
**II**. the aqueous gel phase and the wax phase are combined with thorough mixing whereby an emulsion is formed;
**III**. optionally the solid phase is added with stirring;
**IV**. finally temperature sensitive ingredients such as antioxidants, fragrances, substances etc. are added
**V**. the finished preparation is packaged.

30. A process according to claim 29 **characterised in that** the pH-value of the mixture is adjusted to 7.5 to 8.5, preferably to 7.7 to 8.2.

31. Use of the cosmetic preparation according to one of claims 1 to 28 for colouring and/or shaping keratinic material.

32. Use according to claim 31 for colouring and/or shaping eyelashes, eyebrows, hair, hair pieces, hair which has gone gray at the temples and/or beard hair.

## Patentansprüche

1. Kosmetische Zubereitung in Form einer O/W-Emulsion, die neben üblichen kosmetischen Inhaltsstoffen eine Wachskomponente, einen Emulgator und ein PVP-Copolymer enthält, wobei das Verhältnis von Emulgator zu PVP Copolymer von 10:1 bis 3:1 ist, und wobei der Emulgator Sorbitan-Olivat ist.

2. Kosmetische Zubereitung nach Anspruch 1, **dadurch gekennzeichnet, dass** die Wachskomponente ein oder mehrere Wachse enthält, die bei Raumtemperatur fest sind und einen Tropfpunkt von 45 bis 200 °C aufweisen.

3. Kosmetische Zubereitung nach Anspruch 1 oder 2, **dadurch gekennzeichnet, dass** die Wachskomponente ein oder mehrere Wachse enthält, die eine Härte von 2 bis 40, bestimmt nach der Methode der Nadelpenetration nach ASTM D5, aufweisen.

4. Kosmetische Zubereitung nach einem der vorhergehenden Ansprüche, **dadurch gekennzeichnet, dass** die Wachskomponente zusätzlich mindestens ein Fett und/oder Öl, mindestens eine Fettsäure, mindestens einen mehrwertigen Alkohol und/oder mindestens ein filmbildendes Agens enthält.

5. Kosmetische Zubereitung nach einem der vorhergehenden Ansprüche, **dadurch gekennzeichnet, dass** eine Fettsäure, die der Fettsäure, die im Emulgator verestert ist, entspricht, als Coemulgator vorhanden ist.

6. Kosmetische Zubereitung nach Anspruch 5, **dadurch gekennzeichnet, dass** der Coemulgator eine mit einer organischen Base teilweise neutralisierte Fettsäure ist.

7. Kosmetische Zubereitung nach Anspruch 6, **dadurch gekennzeichnet, dass** die organische Base Aminomethylpropanol oder Triethanolamin oder eine Mischung daraus ist.

8. Kosmetische Zubereitung nach einem der vorhergehenden Ansprüche, **dadurch gekennzeichnet, dass** der Coemulgator eine teilweise neutralisierte Stearinsäure ist.

9. Kosmetische Zubereitung nach einem der vorhergehenden Ansprüche, **dadurch gekennzeichnet, dass** das Verhältnis von Emulgator zu Coemulgator von 3:1 bis 1:3 ist.

10. Kosmetische Zubereitung nach einem der vorhergehenden Ansprüche, **dadurch gekennzeichnet, dass** das PVP-Copolymer ein PVP/Alken-Copolymer ist.

11. Kosmetische Zubereitung nach einem der vorhergehenden Ansprüche, **dadurch gekennzeichnet, dass** das PVP-Copolymer ein PVP/Hexadecen-Copolymer oder ein PVP/Eicosen-Copolymer oder eine Mischung daraus ist.

12. Kosmetische Zubereitung nach einem der vorhergehenden Ansprüche, **dadurch gekennzeichnet, dass** mindestens ein zweiwertiger oder mehrwertiger Alkohol enthalten ist.

13. Kosmetische Zubereitung nach Anspruch 12, **dadurch gekennzeichnet, dass** der zweiwertige oder mehrwertige Alkohol aus Propandiol, Dipropylenglykol, Tripropylenglykol, Glyzerin, Diglyzerin, Triglyzerin, Butandiolen, Pentaerythrit, Hexandiolen, Sorbitol, Xylitol, Mannitol, Alditol, Pantothenol oder deren Mischungen ausgewählt ist.

14. Kosmetische Zubereitung nach Anspruch 13, **dadurch gekennzeichnet, dass** die Menge an dem mindestens zweiwertigen Alkohol, die verwendet wird, zwischen 0,3 und 10 Gew.-%, bevorzugt zwischen 1 und 5 Gew.-%, bezogen auf die Gesamtmenge der Zubereitung, liegt.

15. Kosmetische Zubereitung nach einem der Ansprüche 4 bis 14, **dadurch gekennzeichnet, dass** das filmbildende Agens aus Polyvinylalkohol, PVP/Alken-Copolymer oder einem funktionalisierten Polyurethan oder Gemischen daraus ausgewählt wird.

16. Kosmetische Zubereitung nach Anspruch 15, **dadurch gekennzeichnet, dass** das funktionalisierte Polyurethan ein hydrophiliertes Polyurethan ist, in das anorganische und/oder organische Säurereste eingebaut sind.

17. Kosmetische Zubereitung nach Anspruch 16, **dadurch gekennzeichnet, dass** die Säurereste Sulfonat-, Sulfat-, Phosphonat-, Phosphat- oder Carboxylatreste sind.

18. Kosmetische Zubereitung nach einem der vorhergehenden Ansprüche 15 bis 17, **dadurch gekennzeichnet, dass** es sich bei dem funktionalisierten Polyurethan um Polyurethan-2 handelt.

19. Kosmetische Zubereitung nach einem der vorhergehenden Ansprüche 14 bis 18, **dadurch gekennzeichnet, dass** das filmbildende Agens in Mengen von 0,1 bis 50 Gew.-%, bevorzugt von 0,3 bis 35 Gew.-% und besonders bevorzugt in Mengen von 1 bis 25 Gew.-%, bezogen auf das Gesamtgewicht der Zubereitung, eingesetzt wird.

20. Kosmetische Zubereitung nach einem der vorhergehenden Ansprüche, **dadurch gekennzeichnet, dass** die wässrige Phase ein Hydrokolloid als gelbildendes Agens enthält.

21. Kosmetische Zubereitung nach Anspruch 20, **dadurch gekennzeichnet, dass** das Hydrokolloid aus Zellulosen und Zellulosederivaten, Stärke und Stärkederivaten, Alginaten, Pektinen, Carrageenanen, Tragant, Naturkautschuk, Polyvinylalkohol, Polyvinylpyrrolidon, Dextran oder Mischungen daraus ausgewählt wird.

22. Kosmetische Zubereitung nach einem der vorhergehenden Ansprüche, **dadurch gekennzeichnet, dass** die wässrige Phase ein vorgequollenes Hydrokolloid ist, das aus Polyvinylalkohol, Hydroxyethylzellulose, Carrageenan oder Tragant ausgewählt wird.

23. Kosmetische Zubereitung nach einem der vorhergehenden Ansprüche, **dadurch gekennzeichnet, dass** die kosmetische Zubereitung zusätzlich eine feinteilige Feststoffphase enthält.

24. Kosmetische Zubereitung nach Anspruch 23, **dadurch gekennzeichnet, dass** die Feststoffphase in einem Verhältnis von zwischen 1 und 30 Gew.-%, bevorzugt zwischen 1 und 20 Gew.-% und besonders bevorzugt zwischen 5 und 15 Gew.-% enthalten ist.

25. Kosmetische Zubereitung nach Anspruch 23 oder 24, **dadurch gekennzeichnet, dass** die feinteilige Feststoffphase anorganische und/oder organische Füllstoffe und/oder feine Fasermaterialien und/oder anorganische und/oder organische Pigmente und deren Mischungen aufweist.

26. Kosmetische Zubereitung nach einem der vorhergehenden Ansprüche, **dadurch gekennzeichnet, dass** zusätzlich ein Steroidderivat oder ein Steroid-enthaltendes Material enthalten ist.

27. Kosmetische Zubereitung nach Anspruch 26, **dadurch gekennzeichnet, dass** Phytosterol oder Stanol in freier oder veresterter Form als Steroidderivat enthalten ist.

28. Kosmetische Zubereitung nach Anspruch 27, **dadurch gekennzeichnet, dass** Oryzanol oder Reiskleiewachs enthalten ist.

29. Verfahren zur Herstellung einer kosmetischen Zubereitung nach einem der Ansprüche 1 bis 28, wobei
I. a) eine wässrige Gelphase durch Vorquellen mindestens eines Hydrokolloids in wässrigem Medium zubereitet wird; wasserlösliche oder hydrophile Inhaltsstoffe können zugefügt werden; b) eine Wachsphase durch Schmelzen mindestens eines Wachses zusammen mit hydrophoben Inhaltsstoffen zubereitet wird, wahlweise wird eine Base zugefügt; danach kann Wasser, dessen Temperatur an die Wachsschmelze angepasst worden ist, hinzugefügt werden; c) wahlweise eine Feststoffphase zubereitet wird, indem Pigmente, Füllstoffe und anderes teilchenförmiges Material mit Wasser vermischt werden;
II. die wässrige Gelphase und die Wachsphase durch gründliches Mischen vereinigt werden, wodurch eine Emulsion entsteht;
III. wahlweise die Feststoffphase unter Rühren zugefügt wird;
IV. schließlich Temperatur-empfindliche Inhaltsstoffe wie Antioxidationsmittel, Duftstoffe, Substanzen etc. hinzugefügt werden
V. die fertige Zubereitung verpackt wird.

30. Verfahren nach Anspruch 29, **dadurch gekennzeichnet, dass** der pH-Wert der Mischung auf 7,5 bis 8,5, bevorzugt auf 7,7 bis 8,2 eingestellt wird.

31. Verwendung der kosmetischen Zubereitung nach einem der vorhergehenden Ansprüche 1 bis 28 zur Färbung und/oder Formung von keratinischem Material.

32. Verwendung nach Anspruch 31 zur Färbung und/oder Formung von Wimpern, Augenbrauen, Haaren, Haarteilen, ergrauten Schläfenhaaren und/oder Barthaaren.

## Revendications

1. Préparation cosmétique, sous la forme d'une émulsion huile/eau, qui en plus des ingrédients cosmétiques habituels contient un composant de cire, un émulsifiant et un copolymère PVP, où le rapport d'émulsifiant sur copolymère PVP est compris entre 10:1 et 3:1, et où l'émulsifiant est l'olivate de sorbitane.

2. Préparation cosmétique selon la revendication 1, **caractérisée en ce que** le composant de cire contient une ou plusieurs cires qui sont solides à température ambiante et présentent une température de liquéfaction comprise entre 45 et 200 °C.

3. Préparation cosmétique selon la revendication 1 ou la revendication 2, **caractérisée en ce que** le composant de cire contient une ou plusieurs cires qui présentent une dureté comprise entre 2 et 40, déterminée en utilisant la méthode de pénétration d'aiguille conformément à la ASTM D5.

4. Préparation cosmétique selon l'une des revendications précédentes, **caractérisée en ce que** le composant de cire contient en outre au moins un corps gras et/ou une huile, au moins un acide gras, au moins un alcool polyvalent et/ou au moins un agent de formation de film.

5. Préparation cosmétique selon l'une des revendications précédentes, **caractérisée en ce qu'**un acide gras qui correspond à l'acide gras estérifié dans l'émulsifiant est présent en tant que co-émulsifiant.

6. Préparation cosmétique selon la revendication 5, **caractérisée en ce que** le co-émulsifiant est un acide gras partiellement neutralisé par une base organique.

7. Préparation cosmétique selon la revendication 6, **caractérisée en ce que** la base organique est l'aminométhylpropanol ou la triéthanolamine ou un mélange de ceux-ci.

8. Préparation cosmétique selon l'une des revendications précédentes, **caractérisée en ce que** le co-émulsifiant est de l'acide stéarique partiellement neutralisé.

9. Préparation cosmétique selon l'une des revendications précédentes, **caractérisée en ce que** le rapport d'émulsifiant sur co-émulsifiant est compris entre 3:1 et 1:3.

10. Préparation cosmétique selon l'une des revendications précédentes, **caractérisée en ce que** le copolymère PVP est un copolymère PVP/alcène.

11. Préparation cosmétique selon l'une des revendications précédentes, **caractérisée en ce que** le copolymère PVP est un copolymère PVP/héxadécène ou un copolymère PVP/eicosène ou un mélange de ceux-ci.

12. Préparation cosmétique selon l'une des revendications précédentes, **caractérisée en ce qu'**au moins un alcool divalent ou polyvalent est inclus.

13. Préparation cosmétique selon la revendication 12, **caractérisée en ce que** l'alcool divalent ou polyvalent est sélectionné à partir du groupe constitué de propanediol, de dipropylèneglycol, de tripropylèneglycol, de glycérine, de diglycérine, de triglycérine, de butanediols, de pentaérythritol, d'hexanediols, de sorbitol, de xylitol, de mannitol, d'alditol, de pantothénol ou de mélanges de ceux-ci.

14. Préparation cosmétique selon la revendication 13, **caractérisée en ce que** la quantité du au moins un alcool divalent utilisé est comprise entre 0,3 et 10 % en masse, de préférence entre 1 et 5 % en masse, par rapport à la masse totale de la préparation.

15. Préparation cosmétique selon l'une quelconque des revendications 4 à 14, **caractérisée en ce que** l'agent de formation de film est sélectionné à partir du groupe constitué d'alcool polyvinylique, de copolymère PVP/alcène ou d'un polyuréthane fonctionnalisé ou de mélanges de ceux-ci.

16. Préparation cosmétique selon la revendication 15, **caractérisée en ce que** le polyuréthane fonctionnalisé est un polyuréthane hydrophilisé dans lequel des résidus d'acide inorganique et/ou organique sont incorporés.

17. Préparation cosmétique selon la revendication 16, **caractérisée en ce que** les résidus d'acide sont des résidus de sulfonate, de sulfate, de phosphonate, de phosphate ou de carboxylate.

18. Préparation cosmétique selon l'une des revendications précédentes 15 à 17, **caractérisée en ce que** le polyuréthane fonctionnalisé est le polyuréthane-2.

19. Préparation cosmétique selon l'une des revendications précédentes 14 à 18, **caractérisée en ce que** l'agent de formation de film est utilisé suivant des proportions de 0,1 à 50 % en masse, de préférence de 0,3 à 35 % en masse et en particulier de préférence suivant des proportions de 1 à 25 % en masse, par rapport à la masse totale de la préparation.

20. Préparation cosmétique selon l'une des revendications précédentes, **caractérisée en ce que** la phase aqueuse contient un hydrocolloïde en tant qu'agent de formation de gel.

21. Préparation cosmétique selon la revendication 20, **caractérisée en ce que** l'hydrocolloïde est sélectionné à partir du groupe constitué de celluloses et de dérivés de celluloses, d'amidon et de dérivés d'amidon, d'alginates, de pectines, de carragénines, de gomme adragante, de caoutchoucs végétaux, d'alcool polyvinylique, de polyvinylpyrrolidone, de dextrane et de mélanges de ceux-ci.

22. Préparation cosmétique selon l'une des revendications précédentes, **caractérisée en ce que** la phase aqueuse est un hydrocolloïde pré-gonflé sélectionné à partir du groupe constitué d'alcool polyvinylique, d'hydroxyéthylcellulose, de carragénine ou de gomme adragante.

23. Préparation cosmétique selon l'une des revendications précédentes, **caractérisée en ce que** la préparation cosmétique contient en outre une phase de solides finement divisés.

24. Préparation cosmétique selon la revendication 23, **caractérisée en ce que** la phase de solides est contenue suivant une proportion de 1 à 30 % en masse, de préférence de 1 à 20 % en masse et en particulier de préférence de 5 à 15 % en masse.

25. Préparation cosmétique selon l'une des revendications 23 ou 24, **caractérisée en ce que** la phase de solides finement divisés comprend des agents de remplissage inorganiques et/ou organiques et/ou des matériaux de fibres fines et/ou des pigments inorganiques et/ou organiques et des mélanges de ceux-ci.

26. Préparation cosmétique selon l'une des revendications précédentes, **caractérisée en ce qu'**un dérivé de stéroïde ou un matériau contenant un stéroïde est en outre inclus.

27. Préparation cosmétique selon la revendication 26, **caractérisée en ce qu'**un phytostérol ou un stanol sous forme libre ou estérifiée est inclus en tant que dérivé de stéroïde.

28. Préparation cosmétique selon la revendication 27, **caractérisée en ce qu'**un oryzanol ou une cire de riz est inclus.

29. Procédé de production d'une préparation cosmétique selon l'une quelconque des revendications 1 à 28, dans lequel
I. a) une phase de gel aqueuse est préparée en faisant pré-gonfler au moins un hydrocolloïde dans un milieu aqueux ; des ingrédients solubles dans l'eau ou hydrophiles peuvent être ajoutés ; b) une phase de cire est préparée en liquéfiant au moins une cire en même temps que des ingrédients hydrophobes, optionnellement une base est ajoutée ; et après cela de l'eau, dont la température a été adaptée à la cire liquéfiée, peut être ajoutée ; c) optionnellement une phase solide est préparée en mélangeant des pigments, des agents de remplissage et une autre matière particulaire avec de l'eau ;
II. la phase de gel aqueuse et la phase de cire sont combinées en les mélangeant vigoureusement, grâce à quoi une émulsion est formée ;
III. optionnellement, la phase solide est ajoutée sous agitation ;
IV. enfin des ingrédients sensibles à la température tels que des anti-oxydants, des parfums, des substances, etc., sont ajoutés.
V. la préparation finie est conditionnée.

30. Procédé selon la revendication 29, **caractérisé en ce que** la valeur de pH du mélange est ajustée à une valeur comprise entre 7,5 et 8,5, de préférence entre 7,7 et 8,2.

31. Utilisation de la préparation cosmétique selon l'une des revendications 1 à 28 dans le but de colorer et/ou de mettre en forme un matériau à base de kératine ou kératinique.

32. Utilisation selon la revendication 31, dans le but de colorer et/ou de mettre en forme les cils, les sourcils, les cheveux, les postiches ou portions de cheveux, les cheveux qui sont devenus gris au niveau des tempes et/ou les poils de barbe.
